# EUROPÄISCHE PATENTSCHRIFT

(11) **EP 1 850 913 B1**
(45) Veröffentlichungstag und Bekanntmachung des Hinweises auf die Patenterteilung: **02.09.2009**
(21) Anmeldenummer: 06706859.3
(22) Anmeldetag: 10.02.2006
(51) Int. Cl.: A61P 31/00, A61K 33/00

(54) **MITTEL ZUR BEHANDLUNG VON INFEKTIONEN**
SUBSTANCE FOR THE TREATMENT OF INFECTIONS
SUBSTANCE POUR LE TRAITEMENT D'INFECTIONS

(30) Priorität: 12.02.2005 DE 102005006462
(43) Veröffentlichungstag der Anmeldung: 07.11.2007
(73) Patentinhaber: Templatech GmbH, 64285 Darmstadt (DE)
(72) Erfinder: KAISER, Dirk, 64859 Eppertshausen (DE); FREYBERG, Mark, Andre, 64287 Darmstadt (DE)
(74) Vertreter: Grünecker, Kinkeldey, Stockmair & Schwanhäusser Anwaltssozietät
(86) Internationale Anmeldenummer: PCT/EP2006/001238
(87) Internationale Veröffentlichungsnummer: WO 2006/084741

(56) Entgegenhaltungen:
- WO-A-00/48940
- WO-A-03/045446
- WO-A-20/05049483

## Beschreibung

Auf der Schleimhaut der äußeren Körperhöhlen/Öffnungen wie Vagina, Mund-. Ohren-, Auge sowie Nasenhöhlen leben normalerweise Keime, die für ein gesundes mikrobielles Mikroklima sogar unerlässlich sind. So leben auf Schleimhäuten der Vagina Milchsäurebakterien, deren Stoffwechselprodukt Milchsäure hilft, durch Ansäuern des umgebenden Milieus Infektionen der Schleimhaut durch pathogene Keime zu verhindern. Trotzdem kann es hier ausgelöst durch die verschiedensten Ursachen zu einem Befall durch pathogene Keime kommen.

Alle in den inneren Körperhöhlen vorkommenden Keime müssen per se als pathologisch betrachtet werden, da die inneren Körperhöhlen des menschlichen Körpers normalerweise frei von Keimen sind.

Infektionen der äußeren und inneren Körperhöhlen sind jedoch aufgrund gestiegener Antibiotikaresistenzen der sie verursachenden Keime oft behandlungsresistent.

Die Ursache für Infektionen beispielsweise der menschlichen Blase liegt in der Regel in der Einwanderung von Bakterien über die Harnröhre in den Blasenraum. Die Harnblase selbst ist gegenüber Keimen zwar relativ unempfindlich, hochproblematisch bei Hamblaseninfektionen ist aber das Risiko des Aufsteigens der Infektion über den Harnleiter in die Niere.

Besonders groß ist diese Problematik bei inkontinenten Patienten (dies sind Patienten, die den Harnfluss nicht kontrollieren können) und älteren Personen, sowie bei Patienten mit traumatischen Veränderungen des zentralen Nervensystems, die zudem oft noch unter Blasenentleerungsstörungen leiden. In vielen Fällen führen diese Konstellationen zu chronischen Harnwegsinfekten, die in der Regel eine Dauermedikation erforderlich machen, was wiederum zu fortschreitenden Resistenzen der sie verursachenden Keime gegen Antibiotika führt und schließlich mit einem Aufsteigen der Infektion bis hin zur Niere verbunden mit Nierenproblemen und letztlich Nierenversagen endet.

Die Behandlung dieser Patienten ist erfahrungsgemäß sehr langwierig und kostenaufwendig. Außerdem wird bei Krankenhauspatienten im Falle von Harnwegsinfektionen die Aufenthaltsdauer wesentlich verlängert, was für den Patienten psychische Belastungen sowie für die Gesellschaft dramatische wirtschaftliche Nachteile mit sich bringt.

Durch Ausschaltung der über die Harnröhre eingewanderten Infektionskeime in die Harnblase reduzieren sich mittelfristig die Gefahr einer Nierenschädigung und langfristig der enorme Behandlungsaufwand. Dies um so deutlicher, wenn verhindert werden kann, dass die Nierenschädigung letztlich immer zu einem Dialysepatienten wenn nicht sogar zu einer Nierentransplantation führt.

Harnwegsinfektionen stehen in ihrer Häufigkeit nach den Atemwegsinfektionen an der zweiten Stelle der Infektionserkrankungen. Innerhalb eines Jahres erleiden mindestens 20 Prozent aller Frauen zwischen 20 und 54 Jahren eine Episode einer Harnwegsinfektion (häufigste Ursache für Arbeitsunfähigkeit bei Frauen). Frauen sind viermal häufiger betroffen als Männer. Im Verlauf von Diabetes II sind Harnwegsinfektionen ebenfalls stark zunehmend.

Harnwegsinfektionen bei Kindern bergen grundsätzlich die Gefahr einer Narbenbildung im Nierenparenchym mit möglichen Spätfolgen im Erwachsenenalter, wie erhöhter Blutdruck, und Nierenversagen. Deshalb sind alle Harnwegsinfektionen bei Kindern dringend behandlungsbedürftig. Die Therapiedauer beträgt normalerweise zwischen sieben und vierzehn Tagen.

Ein sehr großes Problem sind die so genannten nosokomialen Infektionen (Infektionen werden als nosokomial bezeichnet, wenn sie mehr als 48 Stunden nach Aufnahme in das Krankenhaus auftreten und sich bei Aufnahme des Patienten nicht in der Inkubationsphase befanden). 90 Prozent aller Katheterträger entwickeln im Laufe eines Klinikaufenthaltes eine Harnwegsinfektion. Aber auch andere Patientengruppen sind von nosokomialen Infektionen betroffen, wobei die Harnwegsinfektionen ca. 40 % ausmachen.

Bei der üblicherweise durchgeführten Behandlung mit Antibiotika stellt die Resistenzentwicklung der Keime ein großes Probleme dar. Infektionen, die man beherrscht zu haben glaubte, werden durch das Auftreten von multiresistenten Keimen/Stämmen immer wieder zu erneuten Problemen. Diese Multiresistenzen sind insbesondere auf die unkritische Verwendung von Antibiotika zurückzuführen.

Die Fortschritte der modernen Medizin der letzten Jahre gehen aufgrund des gestiegenen Alters und schwererer Grunderkrankungen der Patienten mit einer erhöhten Infektionsgefährdung einher. Im Jahr 1990 lag in deutschen Kliniken der Anteil der Patienten über 60 Jahren bei 30% (3,6 Mio.).

Infektionen gehören heutzutage auch zu den häufigsten Komplikationen in der Behandlung und Pflege älterer Patienten.

Das Risiko, in Pflegeeinrichtungen Infektionen zu erwerben, ist im Alter dreimal so hoch wie in jüngeren Jahren. Ab einem Alter von 65 Jahren ist das Risiko für nosokomiale Infektionen um mehr als 20% erhöht, ab dem 75. Lebensjahr sogar auf ca. 40%. In Einrichtungen für geriatrische Patienten stellen diese Art von Infektionen ein häufig unterschätztes Problem dar. Die Inzidenz wird zwischen 10% und 16% angegeben. Am Häufigsten waren vor allem Infektionen der unteren Atemwege (Bronchitis, Pneumonie) mit 49,7% und der Harnwege mit 33,7% anzutreffen.

An Bedeutung gewonnen haben Problemkeime, wie Antibiotika-resistente Staphylokokken (MRSA). Eine Studie in 395 Altenpflegeeinrichtungen zeigte bei 12% der aufgenommenen Patienten MRSA-Stämme. Patienten mit MRSA befanden sich in einem schlechteren Allgemeinzustand und benötigten einen höheren Pflegeaufwand. Trotz der hohen Rate von MRSA-Trägern lag der Anteil der Patienten, die an einer MRSA-Infektion erkrankten, nur bei 3-4%. Infektionen, die in Pflegeeinrichtungen erworben werden, erhöhen die Kosten (insbesondere Kosten für Antibiotika und gegebenenfalls notwendige/längere Klinikaufenthalte) und führen zu erhöhter Morbidität und Letalität.

Die inkontinenten und pflegebedürftigen älteren Patienten sind in dieser großen Zielgruppe immer mehr anwachsend. Angesichts der stark wachsenden Pflegekosten wird dieser Patientenkreis für eine wirkungsvolle, keiner Resistenzbildung unterliegenden Wirksubstanz/Wirkstofflösung immer wichtiger.

Aus dieser größten Zielgruppe sind auch die Kleinkinder während der Windelzeit zu nennen. Auch hier treten in beachtlichem Umfang Harnwegsinfektionen auf, die eine große Gefahr mit Langzeitproblemen darstellen. Eine Antibiotika-freie Behandlung ist hier besonders wünschenswert.

Generell zählen Frauen zu den Patienten, die sehr häufig unter Harnwegsinfektionen leiden.

Eine weitere Gefahr stellen die nosokomialen Infektionen dar, die bei 40 % der Betroffenen zu Harnwegsinfektionen führen. Gerade Katheter zur Urinabfuhr, beispielsweise nach Operationen, führen häufig zu lang andauernden Harnwegsproblemen.

Chronisch kranke Patienten, im besonderen Patienten mit Diabetes, sind von Harnwegsinfektionen stark betroffen. Mit zunehmendem Alter nimmt der Anteil der männlichen Patienten ebenfalls stark zu, während Frauen aller Altersschichten generell in viel höherer Zahl vertreten sind. Eine weitere Patientengruppe sind insbesondere schwangeren Frauen, die relativ häufig an Harnwegsinfektionen erkranken. Hier werden die traditionellen Behandlungsverfahren teilweise als problematisch angesehen. Die Dichlorsäuren und ihre Herstellung wurden in der nicht vorveröffentlichten internationale Patentanmeldung mit dem Aktenzeichen PCT/EP2004/013212 beschrieben. Ebenfalls beschrieben ist dort die Verwendung der entsprechenden Dichlorsäuren zur Förderung der Wundheilung. Eine Verwendung als Desinfektionsmittel wurde im Allgemeinen beschrieben. Die spezifische Verwendung als Mittel zum Desinfizieren von Körperhöhlen, insbesondere des Urogenitalsystems, der Harnblase, des Augen-, Ohren-, Nasen-, Hals- und Rachenraumes und auch des während einer Operation geöffneten Bauchraumes wird nicht offenbart.

Es war daher Aufgabe der vorliegenden Erfindung, eine Möglichkeit zu finden, Entzündungen in/von Körperhöhlen bekämpfen zu können. Insbesondere sollte es sich um eine Möglichkeit handeln, mit deren Hilfe auch Antibiotika-resistente Keime bekämpft werden können. Dabei sollte die Durchführung der Bekämpfung einfach und sicher in der Handhabung sein.

Überraschenderweise gelingt dies leicht und sicher durch zur Verfügung stellen eines Mittels nach Anspruch 1. Weitere Ausgestaltungen der vorliegenden Erfindungen sind in den nebengeordneten und abhängigen Ansprüchen 2 und 3 dargestellt.

Die erfindungsgemäß verwendbaren Dichlorsäuren sind in der folgenden Tabelle 1 gezeigt. Von diesen Dichlorsäuren stellen die Dichlorsäuren Nr. 1 bis Nr. 3 besonders bevorzugte Ausführungsformen der vorliegenden Erfindung dar.

**Tabelle 1 :**

| **Nr.** | **Formale Oxidationsstufen des Chlor** | **Strukturformel der Säure** | **Strukturformel des Di-Anions** |
|---|---|---|---|
| 1 | +5,+5 | 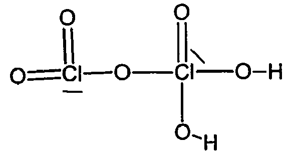 | 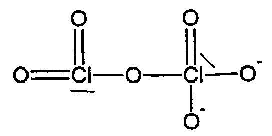 |
| 2 | +6,+4 | 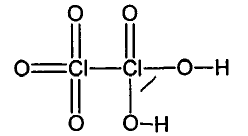 | 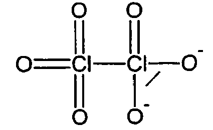 |
| 3 | +5,+5 | 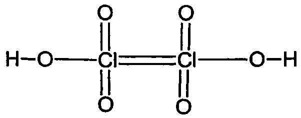 | 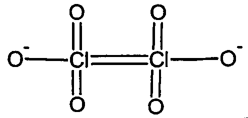 |
| 4 | +5,+3 | 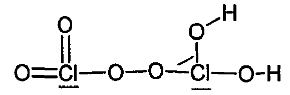 | 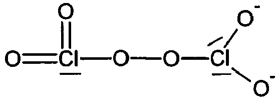 |

Neben den bereits beschriebenen Wertigkeitspaaren +3/+5 (WO 00/48940) und +4/+4 (Bogdanchikov et al.) können die erfindungsgemäß verwendbaren Dichlorsäuren Nr. 1 bis Nr. 3 mit den Wertigkeiten von +6/+4 und +5/+5 für Chlor mit dem erfindungsgemäßen Verfahren zum erstenmal hergestellt werden. Das Anion der Säure der Nr. 4 wird in der WO 00/48940 beschrieben. Das dort beschriebene Herstellungsverfahren funktioniert jedoch nicht.

In der WO 00/48940 wurde postuliert, dass sich aus 2 Molekülen einer reaktiven Chlor-Sauerstoff Spezies (Peroxochlorat) über die Reaktion

2 ⁻OOClO₂ → Cl₂O₆²⁻ + O₂

das Desoxo-Dimere bildet, bei dem die Chloratome in den Oxidationsstufen +3 und +5 vorliegen. Eine arzneimittelrechtlich wünschenswerte Herstellung und Isolierung einer stabilen Verbindung nach Beispiel 6 der WO 00/48940 gelingt jedoch nicht.

Die Entstehung des dimeren Derivats aus 2 Molekülen Peroxochlorat nach der Gleichung

2 ⁻OOClO₂ → Cl₂O₆²⁻ + O₂

ist nämlich nur bei sehr hohen Konzentrationen an Peroxochlorat (etwa ab 2 bis 3 mol/L) zu erwarten. Solche hohen Konzentrationen sind jedoch wegen der hohen Instabilität der Verbindung praktisch nicht darstellbar.

Untersuchungen zeigen jedoch, dass die Umsetzung von Peroxochlorat-lonen O₂ClOO⁻ mit Chlorit-lonen (ClO₂⁻) überraschenderweise direkt zu der Palette der "dimeren" Cl₂O₆²⁻-Spezies führt:

O₂ClOO⁻+ ClO₂⁻ → Cl₂O₆²⁻ -> -> und Isomere

Sofern in der vorliegenden Offenbarung von Anionen die Rede ist, ist die Gegenwart erforderlicher Gegenionen (vor allem in Lösung) eingeschlossen. Es soll mit der Bezeichnung Anionen vor allem zum Ausdruck gebracht werden, dass in Lösung das Dichlorat die stabilere Form gegenüber der protonierten Säure ist.

Die Erfindung betrifft auch das Verfahren zur Herstellung von Zubereitungen, die die Dichlorsäuren bzw. deren Derivate, Anionen und/oder Salze enthalten.

Dadurch, dass man
(a) Chlordioxid mit einer wässrigen oder wasserhaltigen Lösung von Wasserstoffperoxid oder einem anderen Hydroperoxid oder Peroxid bei einem pH-Wert von ≥ 6,5 umsetzt,
(b) den pH-Wert durch Zusatz einer Säure erniedrigt,
(c) die gasförmige freie protonierte Peroxochlorverbindung mit einem gekühlten Gas austreibt und in einer basischen Lösung auffängt,
(d) die aufgefangene Peroxochlorverbindung bei einem pH zwischen 6 und 8, bevorzugt etwa 7 mit einem bis zu 100fachen Überschuss, bevorzugt bis zu 10fachen Überschuss Chlorit inkubiert,
gelingt es auf erstaunlich einfache Art und Weise, die erfindungsgemäß verwendbaren Dichlorsäuren herzustellen.

Die erfindungsgemäß verwendbaren Dichlorsäuren und auch das unter physiologischen pH-Werten vorhandene Anion können daher erfindungsgemäß auch als Gemisch mit Peroxochlorat und Chlorit in Lösung vorliegen. Eine solche, die erfindungsgemäß verwendbaren Dichlorsäuren, Peroxochlorat sowie Chlorit umfassende Lösung zählt daher zu den besonders bevorzugten Ausführungsbeispielen der vorliegenden Erfindung.

In der WO 00/48940 wurden dagegen Chlorit-freie Lösungen erzeugt, in denen die Dichlorsäuren der vorliegenden Erfindung nicht vorhanden sind, oder es wurden chlorithaltige Präparationen erzeugt, die praktisch nur Chlorit enthielten, so dass sie für einen pharmazeutischen Einsatz ungeeignet sind.

Da große Mengen an Chlorit jedoch einer Verwendung der erfindungsgemäß verwendbaren Dichlorsäuren im pharmazeutischen Sektor abträglich sind, ist es insbesondere bevorzugt, wenn in dem Endprodukt der erfindungsgemäßen Lösungen Chlorit in nicht mehr als 20fachem, bevorzugt nicht mehr als 5fachem, und insbesondere nicht mehr als im 3fachen Überschuss in Gewichtsanteilen bezogen auf das Gesamtgewicht der Lösung vorliegt.

Insbesondere liegen die erfindungsgemäß verwendbaren Dichlorsäuren in dieser Lösung in Mengen von etwa 0,1-20 Gew.-%, bevorzugt 3-5 Gew.-% vor, bezogen auf den Gewichtsanteil eingesetztes ClO₂. Der qualitative Nachweis gelingt über Raman-Spektroskopie. Die Durchführung dieser Art von Spektroskopie ist dem Fachmann auf dem Gebiet selbstverständlich. Die erhaltenen Spektrogramme unterscheiden sich deutlich von denen, die mit dem Verfahren der WO 00/48940 erhalten werden. Die Bestimmung des quantitativen Anteils der Dichlorsäure kann über Säure-Base-Titration erfolgen.

Ein weiterer qualitativer Nachweis ist über die Reaktion mit dem Häm-Eisen möglich. In Gegenwart der erfindungsgemäß verwendbaren Dichlorsäuren verläuft der zeitliche Verlauf der Änderung der Intensität der Soret-Bande deutlich anders als mit den Lösungen, die mit dem Verfahren aus der WO 00/48940 erhalten wurden.

Das erfindungsgemäße Verfahren besteht darin, Chlordioxid mit wässrigen oder wasserhaltigem Wasserstoffperoxid oder einem anderen dem Fachmann geläufigen Peroxid oder Hydroperoxid, wie z.B. Peroxocarbonat oder Perborat oder dem Harnstoffaddukt des Wasserstoffperoxids bei einem pH-Wert von 6,5 oder höher, bevorzugt pH 10-12 umzusetzen. Es ist bevorzugt, den pH-Wert auf einem konstanten Wert zu halten.

Darüber hinaus hat es sich überraschenderweise gezeigt, dass die als Zwischenprodukt auftretende Peroxochlorsäure und ihre Anionen und Derivate auch durch die Umsetzung von Chlordioxid mit anderen Oxidationsmitteln, die die Peroxogruppierung enthalten, erhalten werden können.

Die Umsetzung kann in wässrigem Milieu oder in wasserhaltigem Milieu durchgeführt werden. Es können beispielsweise neben Wasser auch mit Wasser mischbare Lösungsmittel vorliegen, wie beispielsweise Alkohole, wie z.B. Alkanole, wie Methanol, Ethanol oder dergleichen, oder Mischungen davon.

Wahlweise kann auch von anderen Chloroxiden ausgegangen werden. So lässt sich beispielsweise Chlormonoxid vorzugsweise in seiner dimeren Form (Cl₂O₂) ebenfalls mit einem Hydroperoxid (vorzugsweise Wasserstoffperoxid) zum gewünschten Produkt umsetzen. Die Umsetzung gelingt im gleichen pH-Gebiet wie beim Chlordioxid angegeben.

Die Reaktionstemperatur kann erhöht werden, beispielsweise bis zu etwa 50°C; bei rein wässrigen Systemen liegt die niedrigste Temperatur bevorzugt bei etwa 0°C. Mit Chlordioxid sollte man allerdings nicht unter +10 Grad Celsius arbeiten, da sich unterhalb dieser Temperatur das Chlordioxid-Gas verflüssigt und es zu Verpuffungen kommen kann. Liegen zusätzliche organische Lösungsmittel und/oder hohe Konzentrationen der beteiligten Reagenzien vor, so können auch niedrigere, d.h. unter dem Gefrierpunkt von Wasser liegende Temperaturen verwendet werden. Bevorzugt wird bei Raumtemperatur gearbeitet.

Das zur Umsetzung benötigte Chlordioxid steht dem Fachmann zur Verfügung und kann in üblicher Weise hergestellt werden. Beispielsweise kann es hergestellt werden durch Reaktion eines Chlorits mit einer Säure (beispielsweise Natriumchlorit mit Schwefelsäure) oder durch Reduktion von Chlorat, beispielsweise mit schwefeliger Säure.

Das so erhaltene Chlordioxid kann gegebenenfalls nach Entfernung von vorhandenen Spuren von Chlor in an sich bekannter Weise (Granstrom, Marvin L.; and Lee, G. Fred, J. Amer. Water Works Assoc. 50, 1453-1466 (1958)) befreit werden.

Sollte das zur ClO₂-Herstellung eingesetzte Chlorit mit Carbonat verunreinigt sein, so entsteht ClO₂ das mit CO₂ verunreinigt ist, und/oder die in der WO00/48940 beschriebenen Kohlensäureaddukte. Zur Absorption des Kohlendioxids sollte der Chlordioxid- und Kohlendioxid-haltige Gasstrom durch eine mit Lauge beschickte Waschflasche geleitet werden. Bei kurzen Kontaktzeiten wird zwar das CO₂, aber nicht das ClO₂ von der Lauge absorbiert. Besser ist es jedoch, die Verunreinigungen an Carbonat durch fraktionierte Kristallisation des eingesetzten Natriumchlorit zu befreien. Eine Verunreinigung des Peroxochlorats mit Carbonat lässt sich leicht im Ramanspektrum erkennen. Anstelle der scharfen Bande bei 1051 cm⁻¹ erhält man eine Doppelbande bei 1069 cm⁻¹ (breit) und die im Rahmen der Erfindung wichtige Bande bei 1051 cm⁻¹ (scharf).

Das Chlordioxid kann mit einem Inertgas, wie Stickstoff oder einem Edelgas, wie Argon, jedoch auch durch Luft oder Sauerstoff zur Reaktion mit der Peroxo-Verbindung oder des Hydroperoxids wie z.B. dem Wasserstoffperoxid oder Percarbonat oder Perborat gefördert werden. Beispielsweise ist es möglich, das Chlordioxid in einem ersten Reaktionsgefäß herzustellen und mit den genannten Gasen oder einem Gemisch daraus in ein zweites Reaktionsgefäß einzuleiten, in dem sich die Peroxo-Verbindung (Peroxid oder Hydroperoxid) in wasserhaltiger oder wässriger Lösung befindet.

Der pH-Wert des Reaktionsgemischs wird durch Zugabe einer Base gleich oder oberhalb 6,5 gehalten. Bevorzugt ist es, den pH-Wert konstant zu halten. Dies kann entweder manuell oder auch automatisch durch ein "pH-Stat"-Gerät erfolgen.

Als Basen können übliche anorganische oder organische Basen, wie Alkalilaugen, beispielsweise Natronlauge oder Kalilauge oder Erdalkalihydroxide, Ammoniak oder organische Basen, wie Stickstoffbasen, dienen. Es können auch die Hydroxide von quaternären Ammoniumsalzen, insbesondere Alkyl-, wie Trialkyl- oder Tetraalkylammoniumhydroxide, oder Zinkhydroxide eingesetzt werden.

Der Gehalt an Hydroperoxid in dem Reaktionsgemisch kann beispielsweise durch potentiometrische Titration mit einer Säure, wie beispielsweise Salzsäure, bestimmt werden.

Die nach dem vorstehend beschriebenen Verfahren erhaltenen Lösungen können als solche oder auch in abgewandelter Form zum Einsatz gebracht werden. Beispielsweise kann überschüssiges Wasserstoffperoxid in üblicher Weise, z.B. mit einer Schwermetallverbindung, wie Braunstein, beseitigt werden. Analog können Überschüsse der anderen Oxidationsmittel beseitigt werden.

Überschüsse an Chlordioxid (ClO₂) können mit H₂O₂ beseitigt werden. Dies sollte möglichst bald geschehen, da ansonsten über

2 ClO₂ + 20H⁻ → ClO₂⁻ + ClO₃⁻ + H₂O

das störende ClO₃⁻ entstünde. Ein Chlorat enthaltendes Produkt sollte jedoch vermieden werden.

Zur Verbesserung der Haltbarkeit des Reaktionsprodukts ist beispielsweise eine Lagerung bei erhöhtem pH-Wert geeignet, beispielsweise bei pH-Wert 10 oder mehr. Die Einstellung dieses pH-Werts kann mit einer geeigneten Base, wie vorstehend zum Herstellungsverfahren beschrieben, vorgenommen werden.

Zur Herstellung von Lösungen, die Dichlorsäuren und/oder die Salze dieser genannten Säuren enthalten ist es überraschenderweise gelungen, die freie Säure HOOClO₂, die Dichlorsäuren bzw. die Peroxochlorsäure bei Erniedrigung des pH-Wertes unter 6, z.B. auf pH-Wert 5 oder weniger aus dem erhaltenen Chloritionen enthaltenden Gemisch mit einem Inertgas, wie einem Edelgas, z.B. Argon oder Stickstoff oder auch den Gasen Sauerstoff oder Luft, auszutreiben und aufzufangen. Es hat sich überraschenderweise gezeigt, dass die Ausbeute enorm gesteigert werden kann, wenn man die Gasstrecke sehr kurz hält und für eine Kühlung des Gasstroms sorgt.

Das bei der Einleitung in Stufe (a) des oben beschriebenen Herstellungsverfahrens entstehende Gemisch enthält zunächst sehr hohe Konzentrationen an Chlorit-Ionen (ClO₂⁻). Der Gehalt an Chlorit kann jedoch durch das "Überleiten" im Gasstrom in eine basische Lösung deutlich reduziert werden. Hierbei werden die Chlorsäuren jeglicher Art als flüchtige Verbindungen in protonierter (neutraler) Form ausgetrieben, die jedoch sehr instabil sind. In der Vorlage befindet sich eine Base, wodurch die Chlorsäuren deprotoniert und die Anionen gebildet werden. Nachdem man die Lösung auf pH 6-8 und nachdem man definierte Mengen an Chlorit beispielsweise in Form von Natriumchlorit zugegeben hat werden dann die Anionen der Dichlorsäuren gebildet.

Sie kann beispielsweise in einer Base, wie einer Alkalimetallbase, Erdalkalimetall- oder Zinkbase oder Stickstoffbase, wie Ammoniak oder einem organischen Amin aufgefangen werden. Es ist aber auch möglich die gasförmig anfallenden Säuren in einer Kühlfalle (z.B. bei Temperaturen von -100 bis -190°C) auszufrieren.

Als Gegenionen kommen alle Metallkationen und organische Kationen, wie diejenigen von Stickstoffbasen, insbesondere quaternäre Ammoniumsalze, in Frage. Welche Kationen besonders geeignet sind, ergibt sich aus dem jeweiligen Verwendungszweck. Für pharmazeutische Anwendungen sind insbesondere Erdalkali- oder Alkalimetalle, vorzugsweise Na⁺ oder K⁺, oder Zn²⁺ bevorzugt, bei technischen Anwendungen können auch organische Kationen, wie Kationen von Stickstoffbasen, insbesondere Alkylammoniumkationen, wie Trialkylammoniumkationen, oder vor allem quaternäre Ammoniumkationen eingesetzt werden.

Es ist zweckmäßig und bevorzugt, die Säure sowie die Salze unter Ausschluss von Licht zu lagern und daraus wässrige Lösungen mit hohen pH-Werten herzustellen, z.B. mit pH-Werten von 10, 11 oder 12 und darüber, insbesondere im Bereich von pH 10 bis pH 13, um lange Lagerfähigkeiten zu erzielen. Aus derartigen Lösungen kann je nach Bedarf die freie Säure, wie vorstehend beschrieben, wieder erhalten und gegebenenfalls in Lösungen mit gewünschtem pH-Wert bzw. in Salze überführt werden.

Die vorliegende Erfindung betrifft ebenfalls pharmazeutische Präparate und Zubereitungen, die als Wirkstoff die erfindungsgemäßen Dichlorsäuren, deren Anionen, Derivate oder Salze umfassen und insbesondere zur Behandlung der eingangs genannten Entzündungen/Infektionen verwendet werden können. Die Präparate enthalten den Wirkstoff alleine oder vorzugsweise zusammen mit einem oder mehreren pharmazeutisch anwendbaren Trägermaterialien oder Lösungsmitteln (physiologische Kochsalzlösung, Ringerlösung etc.). Die Dosierung des Wirkstoffs hängt insbesondere von der Lokalisation der zu behandelnden Entzündung (Blase, Auge, Bauchraum), sowie von Spezies, Alter, Gewicht und individuellem Zustand, individuellen pharmakokinetischen Gegebenheiten sowie der Applikationsweise, der -dauer und den -intervallen ab.

In einer bevorzugten Ausführungsform kann eine 0,025 bis 1 M Lösung eines Dichlorsäuresalzes und/oder eines Salzes von dessen Derivaten in bidestilliertem Wasser bei einem pH gleich oder > 10, vorzugsweise 10 bis 13, insbesondere 12,5, gelöst werden. Diese Lösung/Zubereitung wird unmittelbar vor der Verabreichung mit Kochsalz, Natrium- oder Kaliumbicarbonat, Phosphatpuffer und bidestilliertem Wasser zur Isotonie auf Konzentrationen von ca. 0,01 - 5 mM verdünnt und auf einen annähernd physiologischen pH-Wert eingestellt. Die endgültige zu wählende Konzentration ist hierbei verwendungsabhängig.

Die Anionen der erfindungsgemäß verwendbaren Dichlorsäuren sind stabil, die Säuren selber zerfallen relativ schnell, sodass nach ca. 14 Tagen die Wirksamkeit nachlässt. Eine Arzneiwirkstoffstabilisierung kann deswegen primär über den pH-Wert erreicht werden. Die Wirkstofflösung kann zur Verbesserung der Verträglichkeit unmittelbar vor Gebrauch durch eine Pufferverdünnung auf einen annähernd physiologischen Wert abgesenkt werden. Dies reicht für die Entfaltung der pharmakologischen Wirkung im ganzen Körper aus, denn diese Wirkung beruht nicht auf der Rezeptor-Liganden-Interaktion eines konventionellen Pharmakons, sondern sie ist, wie bereits dargelegt, mit einer schnell und irreversibel ablaufenden Oxidationsreaktion verknüpft. Deren pharmakologische Wirkung hält an, solange die Zelle und/oder ihre chemisch veränderten Strukturen erhalten bleiben, ist also nicht nach Abdiffundieren einer Wirksubstanz von einem Rezeptor beendet.

Die Art und Weise wie dieses Mittel angewendet wird, um die Entzündung zu behandeln, ist dem Fachmann bekannt. Es bestehen hier abhängig vom Ort der Erkrankung vielfältige Möglichkeiten. Da das erfindungsgemäße Mittel eine Lösung ist, bietet sich eine Spülung als leichteste Möglichkeit dar. Hierzu wird der Fachmann beispielsweise eine Katheder verwenden. Im Falle einer Infektion des Urogenitalsystems, insbesondere der Blase, kommt die Instillation bevorzugt zum Einsatz.

Beim Einsatz zur Verhinderung/Prophylaxe von Infektionen im Bauchraum, wie sie beispielsweise als Komplikationen bei Operationen am Magen/Darm-Trakt häufig auftreten wird die Lösung vor dem Verschluss der Bauchdecke in die offene Wunde gegeben und verbleibt dort ("desinfizierendes Depot"). Sie wird dort einfach "abgebaut", sodass ein Spülen im klassischen Sinne hier nicht stattfinden muß.

Die Vorteile des erfindungsgemäßen Mittels liegen auch insbesondere darin, dass eine Resistenz-Entwicklung nicht stattfindet/nicht stattfinden kann.

Außerdem war es völlig überraschend, dass das erfindungsgemäße Mittel neben der extrem guten Wirksamkeit auch keinerlei Unverträglichkeitsreaktion zeigt und keinerlei allergische Reaktionen beobachtet wurden.

Die Instillation ist erfindungsgemäß das tropfenweise Einbringen von flüssigen Medikamenten in den Organismus (Hohlorgan, Körperhöhlen, Körperöffnungen) Bei der Blaseninstillation (stilla = Tropfen) handelt es sich um eine chemotherapeutische Behandlung einer Harnwegsinfektion. Sie kann mittels Spritze oder Applikator beim liegenden Katheter (bzw. vorausgegangenem Katheterisieren) vorgenommen werden.

Ganz allgemein ist das erfindungsgemäße Mittel hervorragend zur Desinfektion beispielsweise durch Nasen-, Ohrspülungen bzw. Spülung beliebiger Körperhöhlen oder auch durch Gabe in Form eines "desinfizierendes Depots" geeignet (z.B. Bauchraum).

Weiterhin ist das erfindungsgemäße Mittel auch hervorragend geeignet, herkömmliche Therapien zur Behandlung der Infektionen von Körperhöhlen zu unterstützen, wie beispielsweise Blasenimplantate wie sie in der Deutschen Patentanmeldung WO 2004/034930 A2 offenbart sind.

Die folgenden Beispiel beschreiben die Erfindung ohne dass diese als die Erfindung einschränkend verstanden werden sollen.

### Beispiel 1

### Herstellung der Dichlorsäuren

Zu einer Lösung von 100 g wasserfreiem Natriumchlorit in 200 mL Wasser gibt man unter Rühren vorsichtig tropfenweise Schwefelsäure (96%-ig) zu. Mit einem kräftigen Gasstrom (Ar, N₂ oder O₂ bzw. CO₂-freie Luft) wird das entstehende Chlordioxid ausgetrieben. Der Gasstrom muss so stark sein, dass der Gehalt an ClO2 nicht über 5 Prozent steigt (Explosionsgefahr). Der ClO₂-haltige Gasstrom wird, um elementares Chlor abzufangen, über drei hintereinander geschaltete Waschflaschen, die mit je 30 mL einer 2 M NaClO₂-Lösung vom pH 11 beschickt sind, in eine Lösung von 15 mL 30%-igem Wasserstoffperoxid in 35 mL Wasser, die zuvor durch Zugabe von 4M Natronlauge auf pH 12 gebracht ist, eingeleitet. An Stelle von Wasserstoffperoxid kann auch eine Lösung von Natriumperborat oder Natriumpercarbonat oder einer anderen Peroxoverbindung wie z.B. das H2O2-Addukt des Harnstoffs verwendet werden. Während der Gaseinleitung wird der pH-Wert mit einer Glaselektrode kontrolliert. Durch Zugabe von 4M NaOH wird der pH-Wert im Verlauf der Reaktion bei 12 gehalten. Das vorgelegte Hydroperoxid bzw. die vorgelegte Peroxoverbindung ist verbraucht, wenn die Gaseinleitung zu einer bleibenden Gelbfärbung führt. Mit einem Tropfen der Lösung des Oxidationsmittels (z.B. H₂O₂) wird die gelbe Lösung anschließend wieder entfärbt.

Die reaktives Chlor enthaltende Lösung wird unter Rühren zu einer Lösung von 500 g Zitronensäure in 3 Liter Wasser getropft, die zuvor mit 2 M Natronlauge auf pH 4,5 eingestellt ist. Während der Zugabe wird die gebildete reaktive Chlorverbindung mit einem kräftigen Gasstrom (N₂ oder O₂) ausgetrieben. Der Gasstrom ist vorzugsweise zu kühlen. Die Schlauchverbindungen sollten möglichst kurz sein. Das Gas wird beispielsweise in drei hintereinandergeschalteten Waschflaschen, die mit je 50 mL 0,1 M NaOH beschickt sind, aufgefangen.

Die Inhalte der Waschflaschen werden vereinigt und bei pH > 10 gehalten.

Zur Bildung der erfindungsgemäß verwendbaren Dichlorsäuren wird der pH auf 7 mit beispielsweise Salzsäure eingestellt, und es wird ein 10facher molarer Überschuß an Natriumchlorit zugegeben.

Der Gesamtgehalt an reaktiven Chlor Anionen wird durch potentiometrische Titration mit 0,1 M HCl in einer dem Fachmann wohl bekannten Weise bestimmt.

Die gebildeten Dichlorsäuren liegen in Lösung in Gemisch mit einer definierten Menge an Chlorit sowie weiteren reaktiven Chlorverbindungen vor.

Das Vorhandensein der Dichlorsäuren wird mit Ramanspektroskopie nachgewiesen.

### Beispiel 2

### Herstellung des erfindungsgemäßen Arzneimittel, Dosierung und Anwendung:

Das erfindungsgemäße Arzneimittel wird durch Verdünnen der Lösung aus Beispiel 1 in einem pharmazeutisch geeigneten Träger (z.B. 0,9% NaCl-Lösung, Ringer-Lösung, Phosphatpuffer etc.) vorzugsweise in einem Verhältnis 1:100 bis 1:5.000 hergestellt.

Dabei können beliebige pharmazeutisch annehmbare Hilfsstoffe zugesetzt werden.

### Beispiel 3

### Behandlung therapieresistenter Blaseninfektionen

Behandlung von Patienten mit neurogenen Blasenentleerungsstörungen z. B durch Querschnittslähmungen mit ausgetestetem, multiresistenten Keimspektrum.

Die Behandlung dieser Patienten erfolgt durch Instillation der Lösung über einen suprapubischen Dauerkatheder. Dabei werden 2x täglich 50 mL einer 1 : 2000 verdünnten Lösung instilliert und für etwa 30 Minuten in der Blase belassen. Danach wird die Spüllösung über den liegenden Katheder wieder abgelassen. Dauer, Konzentration und Volumen können jedoch variieren und werden vom Arzt abhängig von der Schwere der Erkrankung und dem allgemeinen Zustand des Patienten und anderer Faktoren festgesetzt.

Die täglichen Spülvorgänge werden nach Ermessen des Arztes über einen Zeitraum von 5 - 7 Tagen wiederholt. Mittels Austestung und Cytoskopie wird der Heilungsverlauf der Krankheit kontrolliert und die Therapie fortgeführt, bis keine Infektion mehr nachweisbar ist.

## Patentansprüche

1. Verwendung von Dichlorsäuren, die hergestellt werden, in dem man
(a) Chlordioxid mit einer wässrigen oder wasserhaltigen Lösung von Wasserstoffperoxid oder einem anderen Hydroperoxid oder Peroxid bei einem pH-Wert von >=6,5 umsetzt,
(b) den pH-Wert durch Zusatz einer Säure auf 3 bis 6 erniedrigt,
(c) die gasförmige freie Peroxochlorverbindung mit einem gekühlten Gas austreibt und in einer basischen Lösung mit einem pH-Wert von >10 auffängt, und
(d) die aufgefangene Peroxochlorverbindung mit einem bis zu 100fachen Überschuss Chlorit bei einem pH-Wert von 6 bis 8 inkubiert,
oder deren physiologisch verträgliche Derivate, Anionen, oder Salze zur Herstellung eines Medikamentes und/oder einer pharmazeutischen Zubereitung zur antiinfektiösen Behandlung von Körperhöhlen.

2. Verwendung nach Anspruch 1, **dadurch gekennzeichnet, dass** die Körperhöhle Bauchhöhle, Nasen- oder Nasennebenhöhle, Ohr, Mundhöhle oder Harnblase ist.

3. Verwendung nach Anspruch 1 oder 2, **dadurch gekennzeichnet, dass** die Behandlung in Form einer Spülung oder einer Depotmaßnahme durchgeführt wird.

4. Verwendung nach einem der Ansprüche 1 bis 3, **dadurch gekennzeichnet, dass** die als Wirkstoff verwendeten Dichlorsäuren die Bruttoformel H₂Cl₂O₆ und die Strukturformel der Anionen 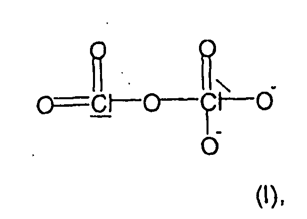 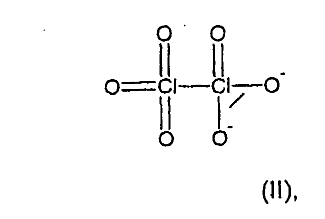 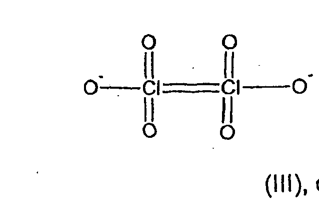 oder 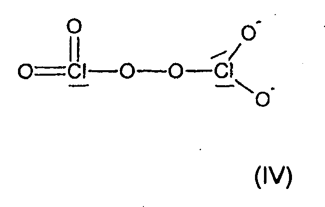 aufweisen.

5. Verwendung nach Anspruch 4, wobei die Strukturformeln der Anionen die Strukturformeln I bis III aufweisen.

6. Verwendung nach einem der vorstehenden Ansprüche, wobei die wässrige Lösung eine Konzentration an Dichlorsäuren von mindestens 0,01 M aufweist.

7. Verwendung nach Anspruch 6, wobei die wässrige Lösung eine Konzentration von mindestens 0,05 M aufweist.

8. Verwendung nach Anspruch 6, wobei die wässrige Lösung eine Konzentration von mindestens 0,075 M aufweist.

9. Verwendung nach Anspruch 6, wobei die wässrige Lösung eine Konzentration von mindestens 0,1 M aufweist.

10. Verwendung nach Anspruch 6, wobei die wässrige Lösung eine Konzentration von mindestens 0,5 M aufweist.

11. Verwendung nach einem der vorhergehenden Ansprüche, wobei die Dichlorsäuren in Form ihrer Alkalimetall-, Erdalkalimetall-, Zink-, Ammoniak- und/oder Aminsalze oder deren Derivate vorliegen.

12. Verwendung nach einem der vorhergehenden Ansprüche, **dadurch gekennzeichnet, dass** man die freie Dichlorsäure oder deren Derivate in Stufe (c) in einer Kühlfalle auffängt.

13. Verwendung nach einem der vorhergehenden Ansprüche, **dadurch gekennzeichnet, dass** man die freie Dichlorsäure oder deren Derivate aus Stufe (d) in eine wässrige alkalische Lösung einleitet.

14. Verwendung nach einem der vorhergehenden Ansprüche, **dadurch gekennzeichnet, dass** man in der basischen Lösung aus Stufe (c) als Base eine Alkalimetall-, Erdalkalimetall-, Zink- oder Stickstoffbase oder ein Hydroxid eines quaternären Ammoniumsalzes verwendet.

15. Verwendung nach einem der vorhergehenden Ansprüche, **dadurch gekennzeichnet, dass** man die aus Stufe (d) erhaltenen Lösungen durch Erhöhen des pH-Wertes stabilisiert.

## Claims

1. Use of dichloric acids, which are produced by
a) reacting chlorine dioxide with an aqueous or water-containing solution of hydrogen peroxide or another hydroperoxide or peroxide at a pH-value of >=6.5,
b) reducing the pH-value to 3 to 6 by adding an acid,
c) expelling the gaseous free peroxochlorine compound with a cooled gas and capturing the same in a basic solution with a pH-value of >10, and
d) incubating the captured peroxochlorine compound with a chlorite excess of up to 100-fold at a pH-value of 6 to 8,
or their physiologically compatible derivates, anions, or salts to produce a medicament and/or a pharmaceutical preparation for the anti-infectious treatment of body cavities.

2. Use according to claim 1, **characterised in that** the body cavity is the abdominal cavity, nasal or near-nasal cavity, aural or buccal cavity or bladder.

3. Use according to claim 1 or 2, **characterised in that** the treatment takes the form of flushing or deposition.

4. Use according to one of claims 1 to 3, **characterised in that** the dichloric acids used as an active ingredient have the total formula H₂Cl₂O₆ and the structural formula of the anions 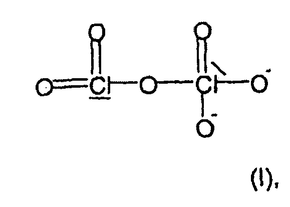 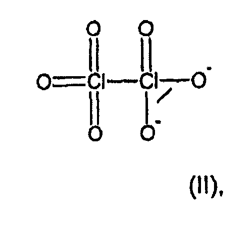 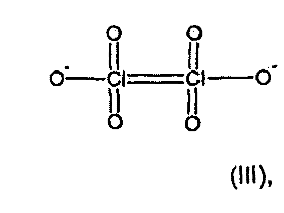 or

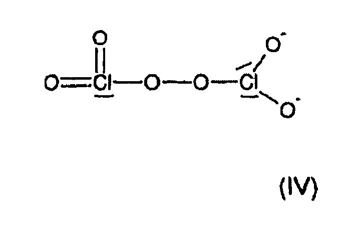

5. Use according to claim 4, wherein the structural formulae of the anions have the structural formulae I to III.

6. Use according to one of the preceding claims, wherein the aqueous solution has a concentration of dichloric acids of at least 0.01 M.

7. Use according to claim 6, wherein the aqueous solution has a concentration of at least 0.05 M.

8. Use according to claim 6, wherein the aqueous solution has a concentration of at least 0.075 M.

9. Use according to claim 6, wherein the aqueous solution has a concentration of at least 0.1 M.

10. Use according to claim 6, wherein the aqueous solution has a concentration of at least 0.5 M.

11. Use according to one of the preceding claims, wherein the dichloric acids are present in the form of their alkali metal-, alkaline earth metal-, zinc-, ammonia- and/or amino salts or their derivatives.

12. Use according to one of the preceding claims, **characterised in that** the free dichloric acid or its derivatives are captured in stage (c) in a cooling trap.

13. Use according to one of the preceding claims, **characterised in that** the free dichloric acid or its derivatives from stage (d) into an alkaline aqueous solution.

14. Use according to one of the preceding claims, **characterised in that** in the basic solution from stage (c), an alkali metal-, alkaline earth metal-, zinc- or nitrogen base or a hydroxide of a quaternary ammonium salt are used as a base.

15. Use according to one of the preceding claims, **characterised in that** the solutions obtained from stage (d) are stabilised by increasing the pH-value.

## Revendications

1. Utilisation d'acides dichlorés produits par
(a) conversion de dioxyde de chlore avec une solution aqueuse ou contenant de l'eau de peroxyde d'hydrogène ou d'un autre hydroperoxyde ou peroxyde à un pH ≥ 6,5,
(b) abaissement du pH à une valeur de 3 à 6 par addition d'un acide,
(c) évacuation du composé peroxochloré libre sous forme gazeuse avec un gaz refroidi et récupération dans une solution basique à un pH > 10, et
(d) incubation du composé peroxochloré récupéré avec un excédent allant jusqu'à un facteur 100 de chlorite à un pH de 6 à 8,
ou de leurs dérivés, anions ou sels physiologiquement acceptables, pour la production d'un médicament et/ou d'une préparation pharmaceutique pour le traitement anti-infectieux de cavités corporelles.

2. Utilisation selon la revendication 1, **caractérisée en ce que** la cavité corporelle est la cavité abdominale, les cavités nasales ou les sinus paranasaux, les oreilles, la cavité buccale ou la vessie.

3. Utilisation selon la revendication 1 ou 2, **caractérisée en ce que** le traitement est réalisé sous la forme d'un lavement ou d'une administration à libération contrôlée.

4. Utilisation selon l'une des revendications 1 à 3, **caractérisée en ce que** les acides dichlorés utilisés comme principe actif présentent la formule brute H₂Cl₂O₆ et la formule développée des anions 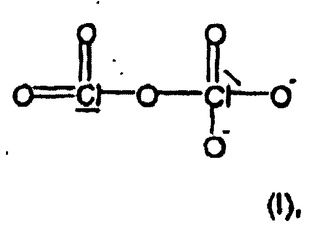 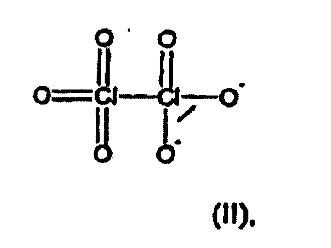 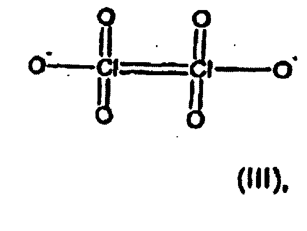 ou

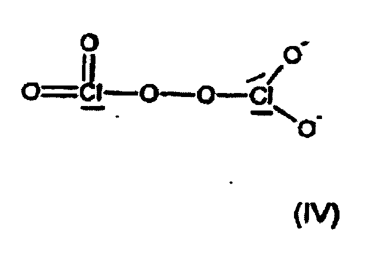

5. Utilisation selon la revendication 4, dans laquelle les formules développées des anions sont les formules développées I à III.

6. Utilisation selon l'une des revendications précédentes, dans laquelle la solution aqueuse présente une concentration en acides dichlorés d'au moins 0,01 M.

7. Utilisation selon la revendication 6, dans laquelle la solution aqueuse présente une concentration d'au moins 0,05 M.

8. Utilisation selon la revendication 6, dans laquelle la solution aqueuse présente une concentration d'au moins 0,075 M.

9. Utilisation selon la revendication 6, dans laquelle la solution aqueuse présente une concentration d'au moins 0,1 M.

10. Utilisation selon la revendication 6, dans laquelle la solution aqueuse présente une concentration d'au moins 0,5 M.

11. Utilisation selon l'une des revendications précédentes, dans laquelle les acides dichlorés se présentent sous la forme de leurs sels de métal alcalin, de sels de métal alcalinoterreux, de sels de zinc, d'ammoniac et/ou d'amine ou de leurs dérivés.

12. Utilisation selon l'une des revendications précédentes, **caractérisée en ce que** l'on récupère l'acide dichloré libre ou ses dérivés à l'étape (c) dans un piège cryogénique.

13. Utilisation selon l'une des revendications précédentes, **caractérisée en ce que** l'on introduit l'acide dichloré libre ou ses dérivés de l'étape (d) dans une solution aqueuse alcaline.

14. Utilisation selon l'une des revendications précédentes, **caractérisée en ce que** l'on utilise comme base dans la solution basique de l'étape (c), une base de métal alcalin, une base de métal alcalinoterreux, une base de zinc ou une base azotée ou un hydroxyde d'un sel d'ammonium quaternaire.

15. Utilisation selon l'une des revendications précédentes, **caractérisée en ce que** l'on stabilise les solutions obtenues à l'étape (d) par augmentation du pH.
